(19) Europäisches Patentamt
European Patent Office
Office européen des brevets

(11) EP 0 771 786 B1

(12) EUROPEAN PATENT SPECIFICATION

(45) Date of publication and mention of the grant of the patent:
**16.02.2000 Bulletin 2000/07**

(51) Int Cl.[7]: **C07C 253/14**

(21) Application number: **96307655.9**

(22) Date of filing: **23.10.1996**

(54) **Preparation of aryl and vinyl nitriles**

Verfahren zur Herstellung von Aryl- und Vinylnitrilen

Procédé de préparation de nitriles d'aryl et vinyl

(84) Designated Contracting States:
**AT BE CH DE DK ES FI FR GB GR IE IT LI LU NL PT SE**

(30) Priority: **31.10.1995 US 8179**

(43) Date of publication of application:
**07.05.1997 Bulletin 1997/19**

(73) Proprietor: **ELI LILLY AND COMPANY
Indianapolis, Indiana 46285 (US)**

(72) Inventors:
- **Anderson, Benjamin Alan
Zionsville, Indiana 46077 (US)**
- **Becke, Lisa Marie
Crawfordsville, Indiana 47933 (US)**

(74) Representative: **Tapping, Kenneth George et al
Lilly Industries Limited
European Patent Operations
Erl Wood Manor
Windlesham Surrey GU20 6PH (GB)**

(56) References cited:
**EP-A- 0 015 427** **EP-A- 0 425 743**
**US-A- 4 211 721**

- **CHEMICAL ABSTRACTS, vol. 121, no. 1, 4 July 1994 Columbus, Ohio, US; abstract no. 8831j, page 937; column 2; XP002025176 & SYNTH. COMMUN., vol. 24, no. 6, 1994, pages 887-890, D.M. TSCHAEN ET AL:**
- **CHEMICAL ABSTRACTS, vol. 76, no. 15, 10 April 1972 Columbus, Ohio, US; abstract no. 85055p, page 357; column 2; XP002025177 & SEKIYU GAKKAI SHI, vol. 14, no. 11, 1971, pages 905-908, N. KOMINAMI ET AL:**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

The preparation and use of aryl and vinyl nitriles are well known in the field of synthetic organic chemistry. The nitrile moiety may serve as a synthetic equivalent for a number of other substituents such as a primary amine or aldehyde. Alternatively, nitriles may be converted into ketones, carboxylic acids, esters or amides. Compounds bearing the nitrile moiety also serve as useful intermediates for the preparation of heterocyclic ring systems.

Aryl nitriles may be prepared by a number of methods well known in the art. One strategy is the transformation of a carboxylic acid, amide or ester into the corresponding nitrile when appropriate substrates are readily available and if no other sensitive functionality in the molecule precludes subjecting the substrate to the necessary reaction conditions. The electrophilic aromatic addition of chlorosulfonylisocyanate to an appropriate substrate may be employed for the preparation of aryl nitriles, but positional isomers are frequently possible and other functionality in the molecule many times complicates these reactions. The Sandmeyer reaction provides nitriles by diazotization of an appropriate aniline followed by displacement of the diazonium salt with cuprous cyanide. This methodology is limited by the requirement that the diazotization step be performed in strong acid, the potential for explosive decomposition of the intermediate diazonium salt, and disposal issues associated with stoichiometric or excess copper. Direct displacements of aryl halides by cuprous cyanide at high temperature are well known in the art but yields are compromised by chelation of the product by the waste copper salts (Ellis, G.P., et *al., Chem. Rev.,* **87,** 779 (1987)) and disposal and environmental issues associated with the use of copper.

Tschaen, D.M. et.al., *Synth. Commun.,* **1994,** 24(6). 887-90 describes a method for the conversion of aryl bromides to aryl cyanides, involving the treatment of the aryl halide with zinc cyanide in the presence of a catalytic amount of palladium(0). The amount of zinc cyanide required for the reaction is 0.6 molar equivalents (60 mole percent), while catalytic amounts of zinc cyanide along with potassium cyanide showed no reaction.

The preparation of vinyl nitriles is typically accomplished by reaction of a vinyl organometallic reagent, such as the corresponding organolithium or organocuprate, with an electrophilic cyanide source such as cyanogen chloride. These reactions must typically be performed at very low temperatures and the reaction conditions limit the diversity of functionality allowed in the substrate.

Palladium catalyzed cyanation reactions for the preparation of aryl and vinyl nitriles have been reported in the literature. Depending on the substrate, these reactions often proceed slowly and commonly do not go to completion in a reasonable period of time, even when performed at elevated temperatures. A variety of palladium catalysts and additional reagents, such as zinc cyanide, cyanotrimethylsilane, alumina, crown ethers, and excess copper(I) iodide, have been suggested to accelerate these reactions. Many of the reaction conditions which have been disclosed for palladium catalyzed cyanations are substrate specific, and a general method has not yet been reported.

This invention provides an improved process for the preparation of aryl and vinyl nitriles comprising reacting the corresponding aryl and vinyl halide or triflate substrates with a source of cyanide ion, a palladium catalyst, and a catalytic amount of a source of metal ion, selected from the group consisting of copper(I), copper(II), or zinc(II) ion, in a suitable reaction medium. The process of the invention provides aryl and vinyl nitriles in superior yields, at higher reaction rates and generates significantly less waste copper or zinc than comparable methods.

This invention is useful for the transformation generically represented by the following equation:

$$\textbf{R-X} \rightarrow \textbf{R-CN},$$

where R represents a radical characterized by an arrangement of atoms comprising a first $sp^2$-hybridized carbon atom double-bonded to a second $sp^2$-hybridized atom which may be either carbon or nitrogen, said first $sp^2$-hybridized carbon atom bearing a substituent, X, which is selected from iodo, bromo or trifluoromethanesulfonyloxy as illustrated by the formulae:

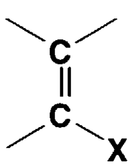

or

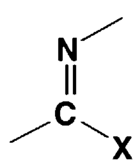

( i ) ( ii ) .

The arrangement of atoms may be part of a chain or ring. The nature of the other groups attached to either the first $sp^2$-hybridized carbon or second $sp^2$-hybridized atom are not critical to the use of the process of the invention so long

as the compound is stable under the conditions employed.

The process of the invention is performed by combining an aryl or vinyl halide or triflate substrate with a source of cyanide ion, a palladium catalyst, and a catalytic amount of a source of copper(I), copper(II), or zinc(II) ion in an appropriate reaction medium. Once the reaction is complete as measured by total consumption of the substrate, the resultant nitrile is isolated by standard extractions and filtrations. If desired, the nitrile product may be further purified by chromatography, crystallization or distillation as appropriate.

The order and manner of combining the reactants are not important and may be varied as a matter of convenience. The substrate, source of cyanide ion, palladium catalyst, and a catalytic amount of a source of copper(I), copper(II), or zinc(II) ion may first be combined and then the reaction medium added. Alternatively, the substrate may first be dissolved in an appropriate reaction medium and this solution added to a mixture of the source of cyanide ion, palladium catalyst, and catalytic amount of a source of copper(I), copper(II), or zinc(II) ion. Also, a solution of the substrate in an appropriate reaction medium may be added to a slurry of the source of cyanide ion, palladium catalyst and copper(I), copper(II), or zinc(II) catalyst in the same reaction medium. Furthermore, a first slurry containing part of the reactants in an appropriate reaction medium may be added to a second slurry of the remaining reactants in an appropriate reaction medium as is desired or convenient. All of these methods are useful for the process of the present invention.

Reactions employing the process of the invention are preferably performed at the reflux temperature of the chosen reaction medium. The reactions may be performed at temperatures below reflux if convenient or desired. The skilled artisan will appreciate that reaction rates typically decrease as temperature is lowered. The overall reaction rate enhancement due to the process of the invention, however, renders lower temperature reactions synthetically useful in many cases.

As discussed previously, compounds useful as substrates for the present invention are aryl and vinyl compounds generically characterized by the arrangement of atoms comprising a first $sp^2$-hybridized carbon atom double-bonded to a second $sp^2$-hybridized atom which may be either carbon or nitrogen as illustrated by the formulae:

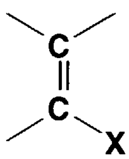

(i) or

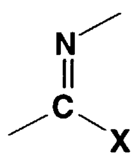

(ii) .

An $sp^2$-hybridized carbon atom is one that uses $sp^2$-hybridized orbitals to form bonds with the three atoms to which it is connected. Likewise, an $sp^2$-hybridized nitrogen atom is one that uses $sp^2$-hybridized orbitals to form bonds with the two atoms to which it is connected. These $sp^2$-hybridized orbitals arise from hybridization of one 2s and two 2p electrons (March, *Advanced Organic Chemistry,* Third Edition (1985), pages 6-9, John Wiley and Sons, New York, NY). The first $sp^2$-hybridized carbon atom bears a substitutent which is selected from iodo, bromo or trifluoromethanesulfonyloxy. It is this substituent which is displaced to give the corresponding aryl and vinyl nitrile products afforded by the process of the invention.

The skilled artisan will appreciate that several types of organic molecules contain the requisite $sp^2$-hybridized atoms in the configuration required to be useful as substrates for the process of the invention. The class of organic molecules known as alkenes, for example, is distinguished by the carbon-carbon double bond. This carbon-carbon double bond is constructed from two $sp^2$-hybridized carbon atoms bonded to each other (Morrison and Boyd, *Organic Chemistry,* Sixth Edition (1992), Section 8.2, pages 273-275, Prentice Hall, Englewood Cliffs, NJ). Alkenes in which at least one of the $sp^2$-hybridized carbons bears a substituent X as defined *supra,* correspond to the arrangement of atoms of formula (i) and are therefore useful substrates for the preparation of vinyl nitriles by the process of the invention. The alkene double bond may be part of a chain or ring. When part of a chain, the double bond may be terminal or within the chain. When part of a ring, the double bond may be exocyclic or incorporated into the ring system. The chain or ring system of which the double bond is part may be further substituted so long as the requirements described *supra* are met.

Another class of compounds which correspond to the arrangement of atoms of formula (i) are those compounds which contain at least one benzene ring. Benzene rings are characterized by six $sp^2$-hybridized carbon atoms bonded together into a ring (Morrison and Boyd, *ibid.,* Section 14.8, pages 501-503). Compounds which incorporate at least one benzene ring into their structure, where at least one of the $sp^2$-hybridized carbons bears a substituent X as defined *supra,* correspond to the arrangement of atoms of formula (i) and are therefore useful substrates for the preparation of aryl nitriles by the process of the invention.

Compounds containing the pyridine ring system are illustrative of the arrangement of atoms of formula (ii). The pyridine ring system is characterized by five $sp^2$-hybridized carbon atoms and one $sp^2$-hybridized nitrogen atom bonded together into a ring (Morrison and Boyd, *ibid.,* Section 30.6, pages 1066-1067). Those pyridine ring compounds where an X substituent is attached to the carbon atom adjacent to the nitrogen atom satisfy the requirements of formula (ii). The skilled artisan will also appreciate that pyridine ring compounds where an X substituent is attached to a carbon not adjacent to the nitrogen atom satisfy the requirements of formula (i). As such, compounds which incorporate at least one pyridine ring into their structure, where at least one of the $sp^2$-hybridized carbons bears a substituent X as defined *supra,* are therefore useful substrates for the preparation of aryl nitriles by the process of the invention.

Aryl compounds which are useful substrates for the process of the invention include compounds containing an aromatic ring. These rings may be isolated or fused in the molecule and may be optionally substituted so long as the requirements described *supra* are satisfied. Examples of aromatic rings which may be incorporated into compounds useful as substrates for the process of the invention include benzene, naphthalene, pentalene, indene, azulene, heptalene, biphenylene, indacene, acenaphthylene, fluorene, phenalene, anthracene, fluoranthene, acephenanthrylene, aceanthrylene, triphenylene, biphenyl, pyrene, chrysene, naphthacene, pleiadene, picene, perylene, pentaphene, pentacene, tetraphenylene, hexaphene, hexacene, rubicene, coronene, trinaphthylene, heptaphene, heptacene, pyranthrene, ovalene, indan, tetralin, acenaphthene, cholanthrene, aceanthrene, acephenanthrene, violanthrene, isoviolanthrene, naphthoperylene, indenoindene, benzocyclooctene and the like.

Aryl compounds which are useful substrates for the process of the invention also include compounds containing a heterocyclic ring. These rings may be isolated or fused in the molecule and may be optionally substituted so long as the requirements described *supra* are satisfied. Examples of heterocyclic rings which may be incorporated into compounds useful as substrates for the process of the invention include furan, thiophene, pyrrole, isopyrroles, pyrazole, imidazole, isoimidazole, triazoles, dithioles, oxathiole, isoxazole, oxazole, thiazole, isothiazole, oxadiazoles, oxatriazoles, dioxazoles, oxathiazole, oxathiole, pyrans, pyrones, dioxins, pyridine, pyridizine, pyrimidine, pyrazine, triazines, oxazines, isoxazines, oxathiazines, oxadiazines, azepine, benzazepines, oxepin, benzoxepins, thiepin, benzthiepins, diazepin, benzdiazepins, benzofuran, isobenzofuran, thionaphthene, isothionaphthene, indole, isoindole, indolenine, pyrindines, pyranopyrazoles, benzpyrazole, benzisoxazole, benzoxazole, anthranil, benzopyrans, benzopyrones, quinoline, isoquinoline, cinnoline, quinazoline, naphthyridine, pyridopyridines, benzoxazines, benzisoxazine, carbazole, xanthene, acridine, purine, and the like.

The skilled artisan will appreciate that if more than one iodo, bromo or trifluoromethanesulfonyloxy substituent is present on an aryl or vinylic position in the substrate, mixtures of mono- and polycyanation products are possible. The exact nature of the product mixture will be dependent upon the reaction conditions and the specific substrate employed.

The source of cyanide ion for the process of this invention is preferably sparingly soluble in the reaction medium. Cyanide ion sources which are completely soluble in the reaction medium, such as tetrabutylammonium cyanide, fail to provide the advantages of the process of the invention. An alkali metal cyanide, such as potassium or sodium cyanide, is especially preferred. The cyanide ion source may be present in from the stoichiometric amount to about 10 equivalents based on the amount of substrate. Preferably from one to two molar equivalents of the cyanide ion source will be present. It is most preferred that about 2 molar equivalents of the cyanide ion source be present. When a solid, it is preferred that the cyanide ion source be ground to a powder for the process of the present invention.

The palladium catalyst for the process of the present invention must be a palladium(0) catalyst such as tris (dibenzylideneacetone)dipalladium(0), tetrakis(triphenylphosphine)palladium(0), and tetrakis(methyldiphenylphosphine)palladium(0). Where the palladium(0) catalyst is complexed to ligands, at least one of the ligands may be bound to an insoluble solid support if desired. Preferably, the palladium catalyst is tetrakis(triphenylphosphine)palladium(0). The palladium catalyst may be present in from about 2 to about 25 mole percent based on the amount of substrate. The amount of palladium catalyst ranging from about 5 to about 10 mole percent is preferred, and about 5 mole percent is most preferred for the process of the present invention. It is also preferred that the palladium(0) catalyst be freshly prepared prior to use. When the palladium(0) catalyst is tetrakis(triphenylphosphine)palladium(0), it is preferred that it be freshly prepared using triphenylphosphine which has been recrystallized from ethanol. The palladium(0) catalyst may alternatively be generated *in situ* from an appropriate source of palladium (II). This alternative is a further embodiment of the present invention.

The metal catalyst for the process of the present invention must be a source of copper(I) ion, such as copper (I) cyanide, a copper(I) halide such as copper(I) iodide or bromide, or potassium dicyanocopper(I); a source of copper (II) ion, such as copper (II) acetate, acetylacetonate, bromide, carbonate, fluoride, hexafluoroacetylacetonate, methoxide, nitrate, perchlorate, sulfate, tetrafluoroborate, trifluoroacetate, trifluoroacetylacetonate, or trifluoromethanesulfonate; or a source of zinc(II) ion, such as zinc(II) cyanide or acetate, or a zinc(II) halide such as zinc(II) fluoride, chloride, bromide, or iodide. When the metal catalyst is copper, the copper catalyst is preferably a source of copper(I) ion. When the metal catalyst is zinc(II), it is preferred that it is zinc(II) iodide. It is especially preferred that the metal catalyst is copper(I) and it is most preferred that the copper catalyst is copper(I) iodide. The metal catalyst may be

present in from about 4 mole percent to about 50 mole percent based on the amount of substrate. The amount of metal catalyst ranging from about 5 to about 25 mole percent is preferred, and about 5 to about 10 mole percent is most preferred for the process of the present invention.

Reaction media useful for the process of the invention must be capable of dissolving a sufficient amount of the substrate for the reaction to proceed. Organic solvents useful as reaction media for the process of this invention include ethers such as tetrahydrofuran, tetrahydropyran, dioxane, diethyl ether and diisopropyl ether, alkyl nitriles such as acetonitrile and propionitrile, and alkyl acetates such as methyl acetate and ethyl acetate. While all of these organic solvents are useful, certain solvents are preferred. Preferred organic solvents include the ethers, especially tetrahydrofuran, and the alkyl nitriles, especially acetonitrile and propionitrile. It is preferred that the reaction media used for the process of the reaction are anhydrous. While it is not necessary to rigorously exclude water for the process of the present invention, excess water will decrease the rate of the reaction. It is also preferred that the reaction medium be deoxygenated by bubbling nitrogen through it prior to use in the process of the invention. Failure to deoxygenate the reaction medium comprises the efficiency of the reaction.

The process may be carried out over a large range of concentrations, from about 0.05 molar to about 1 molar of the substrate, dependent upon the solubility of the particular substrate in the chosen reaction medium. The reaction may also be performed on slurries of the substrate so long as a sufficient amount of the substrate is soluble in the reaction medium for the reaction to proceed. Preferably the process is performed at a concentration from about 0.4 molar to about 1 molar. A concentration of about 0.4 molar to about 0.8 molar is most preferred.

The advantage of the process of the invention is achieved by the addition of a catalytic amount of a source of copper(I), copper(II), or zinc(II) ion to a palladium(0) catalyzed cyanation reaction. This advantage is best demonstrated by comparing cyanation reactions with and without the addition of catalytic metal ion. Typical reaction conditions are described in the following paragraph.

The substrate, 2.0 equivalents of the source of cyanide ion, and 0.05 equivalents of the palladium(0) catalyst are combined in a flask. Parallel reactions are performed with and without 0.1 equivalents of the metal catalyst to demonstrate the advantage obtained by the process of the invention. After the reagents are combined, the reaction flask is placed under vacuum and then refilled with nitrogen. This evacuation/fill cycle is repeated twice, leaving the flask under nitrogen. Nitrogen is bubbled into the desired reaction medium for 5 minutes and then the reaction medium (5.0 mL reaction medium/gm of substrate) is added to the reaction flask. The reaction mixture is then heated to reflux under nitrogen and the reaction monitored by HPLC until complete.

Comparative reactions as described *supra,* using tetrakis(triphenylphosphine)palladium(0) as the palladium (0) catalyst and potassium cyanide as the cyanide ion source were performed on the following series of naphthalene derivatives :

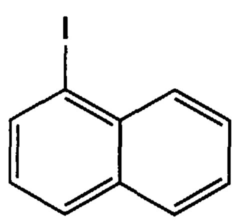

I

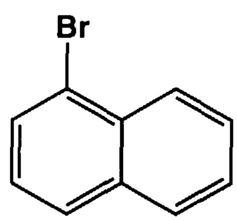

II

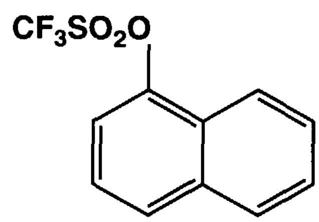

III

The results obtained from these reactions are summarized in Table I.

TABLE I

| | | NO CuI ADDED | | CuI ADDED | |
|---|---|---|---|---|---|
| Substrate | Reaction Medium | Time (hr) | % Nitrile[a] | Time (hr) | % Nitrile[a] |
| I | THF | 1 | 2 | 2 | 100 |
| I | $CH_3CN$ | 8.0 | 12 | 0.25 | 100 |
| II | $CH_3CN$ | 23.0 | 37 | 7.0 | 97 |
| III | $CH_3CN$ | 22.8 | 41 | 0.63 | 100 |

[a]% product relative to remaining starting material

The data presented in Table I clearly demonstrate the advantage to be gained through the use of the method of the

present invention. In all cases, rates of reaction were dramatically increased by addition of a catalytic amount of the copper(I) catalyst to the reaction mixtures. Additionally, in most cases conversions of starting material to product were also dramatically increased.

Comparative reactions as described *supra,* using tetrahydrofuran as the solvent, tetrakis(triphenylphosphine)-palladium(0) as the palladium(0) catalyst and potassium cyanide as the cyanide ion source were performed on 1-iodonaphthalene (I), varying the metal catalyst. The results of these experiments are summarized in Table II.

TABLE II

| CATALYST | AMOUNT | TIME (HR) | % NITRILE |
|---|---|---|---|
| none | none | 1 | 2 |
| CuI | 5 mole % | 2 | 100 |
| CuI | 10 mole % | 1 | 99 |
| CuCN | 10 mole % | 3 | 100 |
| CuOTf | 10 mole % | 4 | 94 |
| $Cu(OTf)_2$ | 10 mole % | 3 | 57 |
| $Cu(OTf)_2$ | 10 mole % | 19 | 100 |
| CuBr | 10 mole % | 3 | 100 |
| $CuBr_2$ | 10 mole % | 6 | 100 |
| $ZnI_2$ | 10 mole % | 2.7 | 94 |

The scope of the present invention can be illustrated further by comparing the results of experiments varying the source of cyanide ion and solvent on the same substrate. (+)-1-Benzoyl-4-dipropylamino-1,2,2a,3,4,5-hexahydrobenz[cd]-indole-6-carbonitrile (IV) is a key intermediate in the preparation of (-)-4-dipropylamino-1,3,4,5-tetrahydrobenz-[cd]indole-6-carboxamide (V).

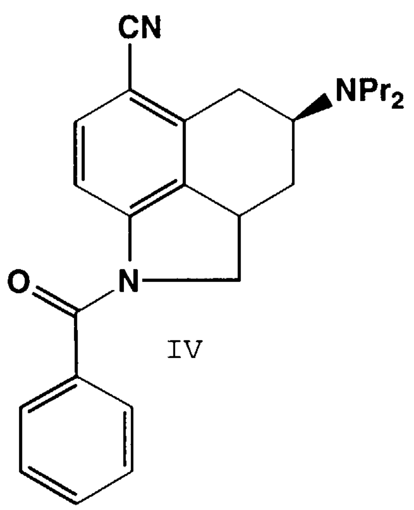

IV

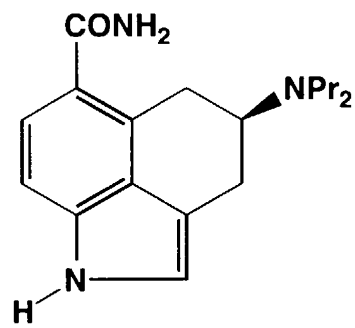

V

The 6-carboxamide (V) is a potent 5-$HT_{1a}$ agonist with therapeutic indications for refractory depression patients and emesis. This intermediate has been prepared from the corresponding 6-bromo-4-dipropylamino-1,2,2a,3,4,5-hexahydrobenz[cd]indole in 76% yield using stoichiometric copper cyanide in N-methyl-2-pyrrolidinone (Martinelli, M.J., *et al., Tetrahedron Letters,* **31**, 7579 (1990)). The 6-bromo and 6-iodo-4-dipropylamino-1,2,2a,3,4,5-hexahydrobenz[cd]indoles were each subjected to the process of the present invention. The results of these experiments are summarized in Table III.

TABLE III

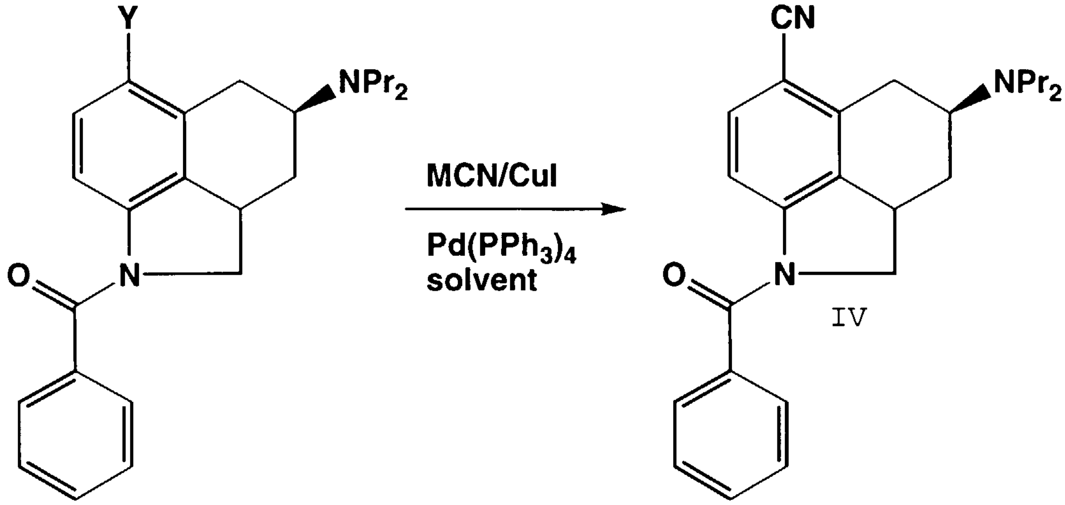


IV

| Y | M | mol % CuI | SOLVENT | TIME (hr) | % IV |
|---|---|---|---|---|---|
| I | K | 0 | THF[1] | 18 | 8 |
| I | K | 10 | THF[1] | 2.4 | 100 |
| I | Na | 10 | THF[1] | 0.5 | 100 |
| I | K | 10 | ACN[2] | 0.5 | 99 |
| I | Na | 10 | ACN[2] | 0.5 | 99 |
| I | K | 10 | EtOAc[3] | 4.7 | 96 |
| I | Na | 10 | EtOAc[3] | 0.6 | 98 |
| Br | Na | 10 | EtCN[4] | 3.1 | 97 |

[1]tetrahydrofuran, [2]acetonitrile, [3]ethyl acetate, [4]propionitrile

The higher yields and reduction in the amount of copper waste generated in these reactions further demonstrate the advantages of the process of the present invention. The results of these experiments also serve to demonstrate the diversity of substrate functionality tolerated by the process of the invention.

Cinoxacin, 1-ethyl-1,4-dihydro-4-oxo[1,2]dioxolo[4,5-g]cinnoline-3-carboxylic acid (VI), is an antibacterial used for urinary tract infections. An intermediate in its synthesis, 1-ethyl-l,4-dihydro-4-oxo[1,2]dioxolo[4,5-g]cinnoline-3-carbonitrile (VII) is prepared by prior art methods from the corresponding 3-bromo compound by reacting it with stoichiometric amounts of copper cyanide. To

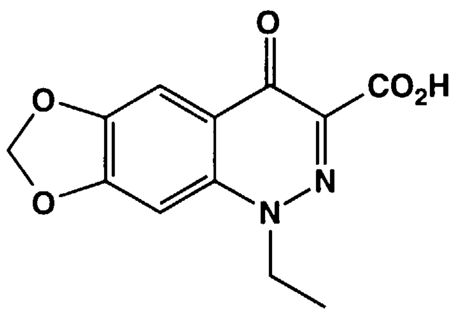

VI

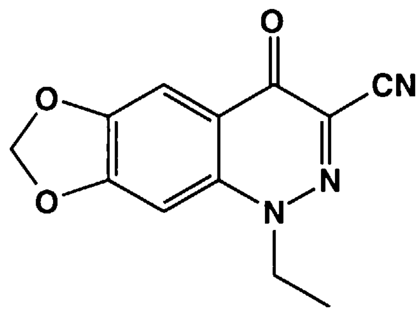

VII

reduce the amount of copper in the final product to acceptable levels, a quantity of the cinoxacin is sacrificed during the prior art synthesis. Subjecting 3-bromo-1-ethyl-1,4-dihydro-4-oxo[l,2]dioxolo[4,5-g]cinnoline to the conditions of the process of the invention using 0.25 equivalents of tetrakis(triphenylphosphine)palladium(0) and 0.50 equivalents of cuprous iodide, 92% of VII was formed after 5 hours in refluxing propionitrile. The process of the invention significantly reduced the amount of copper required for this step in the synthesis of cinoxacin. This reaction further demonstrates the structural diversity tolerated by the process of the present invention.

While a wide variety of structural diversity in the substrate is tolerated by the process of the invention, not all substrates are suitable. The electronic nature of the substrate, for example, can moderate or inhibit the catalytic effect of the present invention. This phenomenon is illustrated by comparing the conversions of substituted iodobenzenes under standard conditions. The results of these experiments are summarized in the following table.

TABLE IV

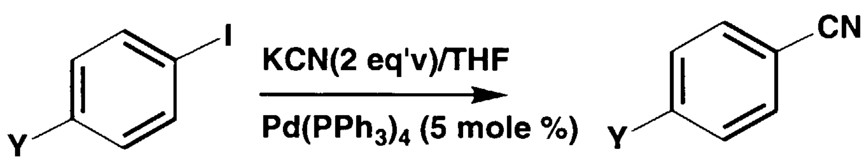


| Y | CuI (mole %) | TIME (hr) | % NITRILE[a] |
|---|---|---|---|
| H | 0 | 4 | 11 |
| H | 10 | 1.5 | 100 |
| $OCH_3$ | 0 | 4 | 14 |
| $OCH_3$ | 10 | 4 | 97 |
| $C(O)CH_3$ | 0 | 4 | 47 |
| $C(O)CH_3$ | 10 | 1 | 100 |
| $NO_2$ | 0 | 1 | 98 |
| $NO_2$ | 10 | 5 | 48 |

[a]As determined by gas chromatography

The beneficial effects of the process of the present invention are lost on the nitro-substituted iodobenzene. This result suggests that nitro-substituted substrates may not be suitable substrates for the process of the present invention.

Assay System

The reactions were assayed by gas chromatography on an HP5890 gas chromatograph with a flame ionization detector and using a 30 m DB-1 (0.25 µm) column (Fisher Scientific). The injection and detection temperatures were both 300°C. The temperature routine began at 50°C for 2 minutes and then the oven temperature increased at a rate of 25°C/minute until it reached a final temperature of 300°C, and was maintained at that temperature for 20 minutes.

EXAMPLE 1

Reaction of 1-iodonaphthalene

A flask containing 1.00 gm (3.94 mMol) 1-iodonaphthalene, 0.23 gm (0.20 mMol) tetrakis(triphenylphosphine) palladium(0), 0.51 gm (7.87 mMol) powdered potassium cyanide, and 0.075 gm (0.39 mMol) copper(I) iodide was placed under vacuum and then charged with nitrogen. This evacuation/fill cycle was repeated twice and the flask left under nitrogen. Nitrogen was bubbled into tetrahydrofuran for 5 minutes and then 5.0 mL were added to the reaction flask. The reaction mixture was then heated to reflux under nitrogen with stirring and the reaction monitored by HPLC. The time course of the reaction is summarized in the following table.

| Time (hr) | % nitrile |
|---|---|
| 0.85 | 74 |
| 2.1 | 96 |
| 3.1 | 99 |

Assay System

The reactions were assayed by HPLC using a Zorbax SB-C8 column (4.6 mm x 25 cm). Peaks were detected with a UV detector at 230 nm. The assays were performed at a flow rate of 1 mL/min with a gradient program. The solvent systems were 1:1 acetonitrile:methanol and 25 mM $NaH_2PO_4$ in water adjusted to pH 2.5 with $H_3PO_4$. The gradient uses 50% of the 1:1 acetonitrile:methanol for 0 to 5 minutes, rises to 60% at 10 minutes, holds steady for 15 minutes, then lowers back to 50% at 20 minutes. Samples for analysis were prepared by adding one drop of the reaction mixture in 6 mL 1:1 methanol:aqueous buffer solution.

EXAMPLE 2

Reaction of 1-bromonaphthalene

A flask containing 1.00 gm (4.83 mMol) 1-bromonaphthalene, 0.28 gm (0.24 mMol) tetrakis(triphenylphosphine)palladium(0), 0.63 gm (9.66 mMol) powdered potassium cyanide, and 0.092 gm (0.48 mMol) copper(I) iodide was placed under vacuum and then charged with nitrogen. This evacuation/fill cycle was repeated twice and the flask left under nitrogen. Nitrogen was bubbled into acetonitrile for 5 minutes and then 5.0 mL were added to the reaction flask. The reaction mixture was then heated to reflux under nitrogen with stirring and the reaction monitored by HPLC. The time course of the reaction is summarized in the following table.

| Time (hr) | % nitrile |
|---|---|
| 0.6 | 27 |
| 3.5 | 87 |
| 4.4 | 92 |
| 5.55 | 95 |
| 7.1 | 97 |
| 23.2 | 100 |

EXAMPLE 3

Reaction of 1-trifluoromethanesulfonyloxynaphthalene

A flask containing 0.50 gm (1.81 mMol) 1-trifluoromethanesulfonyloxynaphthalene, 0.10 gm (0.09 mMol) tetrakis(triphenylphosphine)palladium(0), 0.24 gm (3.62 mMol) powdered potassium cyanide, and 0.34 gm (0.18 mMol) copper(I) iodide was placed under vacuum and then charged with nitrogen. This evacuation/fill cycle was repeated twice and the flask left under nitrogen. Nitrogen was bubbled into acetonitrile for 5 minutes and then 2.5 mL were added to the reaction flask. The reaction mixture was then heated to reflux under nitrogen with stirring and the reaction monitored by HPLC. The reaction was complete within 0.63 hour.

EXAMPLE 4

Reaction of 6-bromo-4-dipropylamino-1,2,2a,3,4,5-hexahydrobenz[cd]indole

A flask containing 0.50 gm (1.13 mMol) 6-bromo-4-dipropylamino-1,2,2a,3,4,5-hexahydrobenz[cd]indole, 0.065 gm (0.057 mMol) tetrakis(triphenylphosphine)palladium(0), 0.11 gm (2.27 mMol) powdered sodium cyanide, and 0.022 gm (0.116 mMol) copper(I) iodide was placed under vacuum and then charged with nitrogen. This evacuation/fill cycle was repeated twice and the flask left under nitrogen. Nitrogen was bubbled into propionitrile for 5 minutes and then 2.5 mL were added to the reaction flask. The reaction mixture was then heated to reflux under nitrogen with stirring and the reaction monitored by HPLC. The time course of the reaction is summarized in the following table.

| Time (hr) | % nitrile |
|---|---|
| 1.0 | 85 |
| 1.6 | 95 |
| 3.1 | 97 |
| 4.0 | 99 |
| 5.2 | 99 |
| 6.4 | 100 |

EXAMPLE 5

Reaction of 6-iodo-4-dipropylamino-1,2,2a,3,4,5-hexahydrobenz[cd]indole in acetonitrile

A flask containing 1.00 gm (2.05 mMol) 6-iodo-4-dipropylamino-1,2,2a,3,4,5-hexahydrobenz[cd]indole, 0.12 gm (0.10 mMol) tetrakis(triphenylphosphine)palladium(0), 0.20 gm (4.10 mMol) powdered sodium cyanide, and 0.039 gm (0.21 mMol) copper(I) iodide was placed under vacuum and then charged with nitrogen. This evacuation/fill cycle was repeated twice and the flask left under nitrogen. Nitrogen was bubbled into acetonitrile for 5 minutes and then 5.0 mL were added to the reaction flask. The reaction mixture was then heated to reflux under nitrogen with stirring and the reaction monitored by HPLC. The reaction was complete within 0.50 hour.

EXAMPLE 6

Reaction of 6-iodo-4-dipropylamino-1,2,2a,3,4,5-hexahydrobenz[cd]indole in ethyl acetate

A flask containing 1.00 gm (2.05 mMol) 6-iodo-4-dipropylamino-1,2,2a,3,4,5-hexahydrobenz[cd]indole, 0.12 gm (0.10 mMol) tetrakis(triphenylphosphine)palladium(0), 0.20 gm (4.10 mMol) powdered sodium cyanide, and 0.039 gm (0.21 mMol) copper(I) iodide was placed under vacuum and then charged with nitrogen. This evacuation/fill cycle was repeated twice and the flask left under nitrogen. Nitrogen was bubbled into ethyl acetate for 5 minutes and then 5.0 mL were added to the reaction flask. The reaction mixture was then heated to reflux under nitrogen with stirring and the reaction monitored by HPLC. The reaction was 98% complete within 0.55 hour and 100% complete at 1.07 hr.

EXAMPLE 7

Reaction of 3-bromo-1-ethyl-1,4-dihydro-4-oxo[l,2]dioxolo[4,5-g]cinnoline

A flask containing 0.20 gm (0.67 mMol) 3-bromo-1-ethyl-1,4-dihydro-4-oxo[1,2]dioxolo[4,5-g]cinnoline, 0.19 gm (0.17 mMol) tetrakis(triphenylphosphine)palladium(0), 0.33 gm (6.73 mMol) powdered sodium cyanide, and 0.064 gm (0.34 mMol) copper(I) iodide was placed under vacuum and then charged with nitrogen. This evacuation/fill cycle was repeated twice and the flask left under nitrogen. Nitrogen was bubbled into propionitrile for 5 minutes and then 10 mL were added to the reaction flask. The reaction mixture was then heated to reflux under nitrogen with stirring and the reaction monitored by HPLC. The time course of the reaction is summarized in the following table.

| Time (hr) | % nitrile |
|---|---|
| 0.67 | 76 |
| 1.93 | 88 |
| 3.12 | 92 |
| 5.0 | 92 |

EXAMPLE 8

Preparation of benzonitrile

A flask containing 1.00 gm (6.4 mMol) bromobenzene, 0.38 gm (0.32 mMol) tetrakis(triphenylphosphine) palladium(0), 0.64 gm (13.12 mMol) powdered sodium cyanide, and 0.12 gm (0.67 mMol) copper(I) iodide is placed under vacuum and then charged with nitrogen. This evacuation/fill cycle is repeated twice and the flask left under nitrogen. Nitrogen is bubbled into tetrahydrofuran for 5 minutes and then 5.0 mL are added to the reaction flask. The reaction mixture is then heated to reflux under nitrogen with stirring and the reaction monitored by HPLC until complete. The reaction mixture is then cooled to room temperature, filtered and the title compound isolated by fractional distillation of the filtrate.

## Claims

1. A process for the transformation :

$$\mathbf{R\text{-}X \rightarrow R\text{-}CN,}$$

comprising reacting the substrate, R-X, with a source of cyanide ion, a palladium(0) catalyst, and a catalytic amount of a metal ion, selected from the group consisting of copper(I), copper(II), or zinc(II) ion, in a suitable reaction medium, where R represents a radical characterized by an arrangement of atoms comprising a first $sp^2$-hybridized carbon atom double-bonded to a second $sp^2$-hybridized atom which may be either carbon or nitrogen, said first $sp^2$-hybridized carbon atom bearing a substituent, X, which is selected from iodo, bromo or trifluoromethanesulfonyloxy.

2. The process of Claim 1 where R is a radical comprising the arrangement of atoms of formula (i):

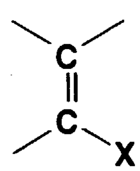

(i) ,

where X is selected from iodo, bromo or trifluoromethanesulfonyloxy.

**3.** The process of Claim 1 where R is a radical comprising the arrangement of atoms of formula (ii):

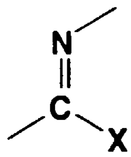

( ii ) ,

where X is selected from iodo, bromo or trifluoromethanesulfonyloxy.

**4.** The process of Claim 1 where the source of cyanide ion is selected from sodium cyanide or potassium cyanide.

**5.** The process of Claim 1 where the palladium(0) catalyst is tetrakis(triphenylphosphine)palladium(0).

**6.** The process of Claim 1 where the metal ion is copper(I) or copper(II).

**7.** The process of Claim 6 where the metal ion is copper(I) and the source of copper(I) ion is copper(I) iodide.

**8.** The process of Claim 1 where the reaction medium is selected from tetrahydrofuran, acetonitrile, propionitrile or ethyl acetate.

**9.** The process of Claim 8 where the reaction medium is tetrahydrofuran.

**10.** The process of Claim 8 where the reaction medium is selected from acetonitrile or proprionitrile.

**Patentansprüche**

**1.** Verfahren zur Umwandlung

$$R\text{-}X \rightarrow R\text{-}CN ,$$

gekennzeichnet durch Umsetzung des Substrats R-X mit einer Cyanidionenquelle, einem Palladium-(0)-katalysator und einer katalytischen Menge eines Metallions, ausgewählt aus der Gruppe, die besteht aus Kupfer-(I)-, Kupfer-(II)- oder Zink-(II)-ion, in einem geeigneten Reaktionsmedium, worin R für einen Rest steht, der durch eine Atomanordnung charakterisiert ist, die ein erstes $sp^2$-hybridisienes Kohlenstoffatom über eine Doppelbindung an ein zweites $sp^2$-hybridisiertes Atom gebunden umfaßt, das entweder Kohlenstoff oder Stickstoff sein kann, wobei das erste $sp^2$-hybridisierte Kohlenstoffatom einen Substituenten X trägt, der ausgewählt ist aus Iod, Brom oder Trifluormethansulfonyloxy.

**2.** Verfahren nach Anspruch 1, worin R für einen Rest steht, der die Atomanordnung der Formel (i) umfaßt

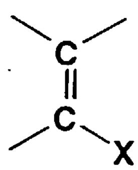

( i ) ,

worin X ausgewählt ist aus Iod, Brom oder Trifluormethansulfonyloxy.

3. Verfahren nach Anspruch 1, worin R für einen Rest steht, der die Atomanordnung der Formel (ii) umfaßt

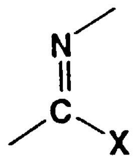

( ii ) ,

worin X ausgewählt ist aus Iod, Brom oder Trifluormethansulfonyloxy.

4. Verfahren nach Anspruch 1, worin die Cyanidionenquelle ausgewählt ist aus Natriumcyanid oder Kaliumcyanid.

5. Verfahren nach Anspruch 1, worin der Palladium-(0)-katalysator Tetrakis(triphenylphosphin)palladium-(0) ist.

6. Verfahren nach Anspruch 1, worin das Metallion Kupfer-(I) oder Kupfer-(II) ist.

7. Verfahren nach Anspruch 6, worin das Metallion Kupfer-(I) und die Kupfer-(I)-ionenquelle Kupfer-(I)-iodid ist.

8. Verfahren nach Anspruch 1, worin das Reaktionsmedium ausgewählt ist aus Tetrahydrofuran, Acetonitril, Propionitril oder Ethylacetat.

9. Verfahren nach Anspruch 8, worin das Reaktionsmedium Tetrahydrofuran ist.

10. Verfahren nach Anspruch 8, worin das Reaktionsmedium ausgewählt ist aus Acetonitril und Propionitril.

## Revendications

1. Procédé pour la transformation:

$$R\text{-}X \rightarrow R\text{-}CN$$

comprenant la mise en réaction du substrat R-X avec une source d'ion cyanure, avec un catalyseur de palladium (0) et avec une quantité catalytique d'un ion métallique choisi parmi le groupe constitué par un ion cuivre(I), un ion cuivre(II) ou un ion zinc(II), dans un milieu de réaction approprié, R représentant un radical caractérisé par un arrangement d'atomes comprenant un premier atome de carbone à hybridation $sp^2$ doublement lié à un second atome à hybridation $sp^2$, qui peut représenter, soit un atome de carbone, soit un atome d'azote, ledit premier atome de carbone à hybridation $sp^2$ portant un substituant X choisi parmi le groupe comprenant un atome d'iode, un atome de brome ou un groupe trifluorométhanesulfonyloxy.

2. Procédé selon la revendication 1, dans lequel R représente un radical comprenant l'arrangement d'atomes répondant à la formule (i):

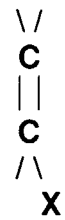

**(i)**

dans laquelle X est choisi parmi le groupe comprenant un atome d'iode, un atome de brome ou un groupe trifluorométhanesulfonyloxy.

**3.** Procédé selon la revendication 1, dans lequel R représente un radical comprenant l'arrangement d'atomes répondant à la formule (ii):

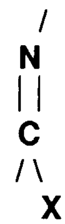

**(ii)**

dans laquelle X est choisi parmi le groupe comprenant un atome d'iode, un atome de brome ou un groupe trifluorométhanesulfonyloxy.

**4.** Procédé selon la revendication 1, dans lequel la source d'ion cyanure est choisie parmi le cyanure de sodium et le cyanure de potassium.

**5.** Procédé selon la revendication 1, dans lequel le catalyseur de palladium(0) est le tétrakis(triphénylphosphine) palladium(0).

**6.** Procédé selon la revendication 1, dans lequel l'ion métallique est le cuivre(I) ou le cuivre(II).

**7.** Procédé selon la revendication 6, dans lequel l'ion métallique est le cuivre(I) et la source de l'ion cuivre(I) est l'iodure de cuivre(I).

**8.** Procédé selon la revendication 1, dans lequel le milieu de réaction est choisi parmi le groupe comprenant le tétrahydrofuranne, l'acétonitrile, le propionitrile ou l'acétate d'éthyle.

**9.** Procédé selon la revendication 8, dans lequel le milieu de réaction est le tétrahydrofuranne.

**10.** Procédé selon la revendication 8, dans lequel le milieu de réaction est choisi parmi l'acétonitrile et le propionitrile.